# FASCICULE DE BREVET EUROPEEN

(11) **EP 4 009 940 B1**
(45) Date de publication et mention de la délivrance du brevet: **30.10.2024**
(21) Numéro de dépôt: 20757653.9
(22) Date de dépôt: 06.08.2020
(51) Int. Cl.: A61K 8/9789, A61Q 19/00

(54) **NOUVELLE UTILISATION COSMÉTIQUE D'UN EXTRAIT D'EPILOBIUM ANGUSTIFOLIUM**
NEUE KOSMETISCHE VERWENDUNG EINES EXTRAKTES VON EPILOBIUM ANGUSTIFOLIUM
NEW COSMETIC USE OF AN EXTRACT OF EPILOBIUM ANGUSTIFOLIUM

(30) Priorité: 08.08.2019 FR 1909079
(43) Date de publication de la demande: 15.06.2022
(73) Titulaire: BASF Beauty Care Solutions France SAS, 69007 Lyon (FR)
(72) Inventeur: ECHARD, Anabelle, 54425 PULNOY (FR); HENRY, Florence, 54425 PULNOY (FR); PELLETIER, Nicolas, 69366 LYON CEDEX (FR); PAIN, Sabine, 69366 LYON CEDEX (FR)
(74) Mandataire: Cabinet Becker et Associés
(86) Numéro de dépôt international: PCT/FR2020/051445
(87) Numéro de publication internationale: WO 2021/023952

(56) Documents cités:
- CA-A1- 2 386 648
- DATABASE GNPD [online] MINTEL; 4 August 2010 (2010-08-04), ANONYMOUS: "Luminizing Black Mask", XP055683370, retrieved from www.gnpd.com Database accession no. 1379982
- INTERNET CITATION, 5 February 2004 (2004-02-05), XP002269178, Retrieved from the Internet <URL:http://www.dweckdata.com/Lectures/SCS_Nov_1992.pdf> [retrieved on 20040205]

## Description

La présente invention est relative à une nouvelle utilisation cosmétique d'un extrait de la plante *Epilobium angustifolium.*

Les pores cutanés constituent les orifices par lesquels s'écoulent les sécrétions des glandes sébacées et des glandes sudoripares. Ils contiennent un follicule pileux et une glande sébacée. Différents facteurs externes tels que le rayonnement UV, la chaleur, la pollution mais aussi une mauvaise hygiène ou un régime alimentaire déséquilibré, et plus simplement le vieillissement cutané, peuvent contribuer à la dilatation des pores, à rendre la peau moins lisse et disgracieuse. Les pores deviennent visibles et l'effet qui en résulte est inesthétique. En outre, des facteurs intrinsèques tels que les hormones androgènes et facteurs de croissance modulent l'activité des glandes sébacées et sudoripares.

L'IGF-1 (Insulin Growth Factor 1) est un facteur de croissance produit par les fibroblastes et les mélanocytes de la peau. Il est essentiel au développement normal de l'épiderme. Son expression diminue avec l'âge. Les kératinocytes expriment le récepteur de l'IGF-1. Il a été montré que l'IGF-1 produit par les fibroblastes dans le derme activait les kératinocytes. Cependant, si trop d'IGF-1 est produit, on note une hyperactivité des kératinocytes qui entraine une différentiation accrue des kératinocytes, et donc une hyperkératinisation de la peau. La peau présente des pores de taille plus grande, donc plus visibles.

De plus, dans la voie de signalisation d'IGF-1, les protéines nommées Insulin Growth Factor Binding Protein (IGFBP), modulent l'activité d'IGF-1. L'IGFBP3 est la protéine majoritaire sécrétée dans les kératinocytes, et de manière générale dans l'épiderme. En se liant à l'IGF-1, l'IGFBP3 bloque la fixation de l'IGF-1 sur son récepteur, et empêche l'hyperprolifération et l'hyperdifférentiation des kératinocytes. L'IGFBP3 participerai ainsi à la diminution de la taille et donc de la visibilité des pores.

En outre, le collagène de type IV est une protéine constitutive des membranes basales, au niveau des jonctions dermo-épidermiques, que l'on retrouve au niveau de la peau mais aussi de ses annexes, en particulier les follicules pileux, les glandes sudoripares et les glandes sébacées. Son expression diminue avec l'âge et est aussi soumis à des facteurs extrinsèques tels que le rayonnement UV qui vont diminuer son expression. Il constitue par conséquent une cible de choix complémentaire pour le développement d'ingrédients cosmétiques actifs sur la réduction de la visibilité des pores cutanés.

Des ingrédients cosmétiques actifs capables de réduire la visibilité des pores existent déjà sur le marché. Mais les domaines de la cosmétique et de la dermopharmacie sont en demande constante d'ingrédients actifs alternatifs de ce type, qui soient stables et procurent un effet durable sur la peau.

La demanderesse a découvert de manière surprenante qu'un extrait *d'Epilobium angustifolium* avait la capacité de diminuer la visibilité des pores de la peau et/ou de prévenir son augmentation.

Des applications cosmétiques de l'extrait selon l'invention sont déjà connus dans l'art antérieur. Ainsi, la demande FR2831440 décrit une activité inhibitrice des phospholipases A2, lipoxygénases et/ou des enzymes de synthèse des prostaglandines par un extrait d'*E*. *angustifolium,* l'extrait possédant un effet sur les rougeurs et les irritations de la peau.

Les demandes JP2003342154 et WO2004016236 divulguent des activités anti-oxidantes d'un extrait d'E. *angustifolium.*

La demande FR2890309 décrit un procédé de soin cosmétique pour atténuer les imperfections cutanées de la peau comprenant l'application de trois compositions successives dont une composition de soin pouvant comprendre un agent apaisant tel qu'un extrait d'*E*. *angustifolium.* Toutefois, cet extrait est décrit dans une longue liste d'agents apaisants.

La demande CA2386648 divulgue une méthode de traitement de la peau comprenant l'administration d'une quantité efficace d'un extrait d'*E*. *angustifolium* en tant qu'agent anti-bactérien contre l'acné.

Enfin, la demande US2016184216 divulgue une composition antipelliculaire comprenant un extrait d'*E*. *angustifolium.* Un effet de l'extrait sur la sécrétion de sébum y est décrit.

Ainsi, aucun des arts antérieurs cités ne décrit ni ne suggère une utilisation d'un extrait d'*E*. *angustifolium* pour diminuer la visibilité des pores de la peau et/ou prévenir son augmentation.

On distingue en effet au sens de l'invention un ingrédient actif sur le sébum, lequel est sécrété par les glandes sébacées et est caractéristique des peaux grasses lorsque présent en excès, d'un ingrédient actif sur les pores de la peau, de façon avantageuse par régulation de l'hyperkératinisation.

Un ingrédient actif peut donc resserrer les pores de la peau sans être actif sur la régulation du sébum, de façon particulière sur l'activité des sébocytes. Il s'agit de 2 effets distincts.

Ainsi, à la connaissance de la demanderesse, aucun art antérieur ne décrit ni ne suggère un effet de maintien et/ou de diminution de la visibilité des pores associé à un extrait d'*E*. *angustifolium.*

La plante *E*. *angustifolium,* aussi nommée épilobe ou willowherb en anglais, est une espèce du genre Chamerion de la famille des Onagraceae que l'on trouve dans l'hémisphère nord de la planète. Cette plante est notamment connue pour entrer dans la fabrication de miels de montagne. Ses feuilles possèdent une activité contre les migraines, les troubles du sommeil lorsqu'elles sont utilisées en tisane. Elles ont aussi des propriétés cicatrisantes.

L'extrait d'*E*. *angustifolium* selon l'invention présente l'avantage de pouvoir être produit facilement à l'échelle industrielle. Il s'agit d'une matière première végétale facilement accessible et renouvelable de façon constante.

L'extrait présente par ailleurs l'avantage d'être toléré par tout type de peaux. Il n'est pas toxique et n'induit pas d'irritations ou réactions allergiques.

Un premier objet de l'invention concerne ainsi l'utilisation cosmétique, non thérapeutique, d'un extrait d'*E*. *angustifolium* pour diminuer la visibilité des pores de la peau et/ou prévenir son augmentation. Un autre objet concerne ladite utilisation dans une composition cosmétique comprenant au moins un excipient cosmétiquement acceptable. Un autre objet concerne encore un procédé de soin cosmétique comprenant l'administration par voie topique ou orale de l'extrait d'*E*. *angustifolium* selon l'invention ou d'une composition cosmétique le comprenant.

Un premier objet de l'invention est relatif à l'utilisation cosmétique, non thérapeutique, d'un extrait d'E. *angustifolium* pour diminuer la visibilité des pores de la peau et/ou prévenir son augmentation.

On entend par « utilisation cosmétique » une utilisation non thérapeutique, non pharmaceutique ni dermatologique, c'est-à-dire qui ne nécessite pas de traitement thérapeutique et destinée à des peaux saines. On entend par « peaux saines » des peaux qualifiées de non pathologiques par un spécialiste du domaine, un dermatologue, c'est à dire ne présentant pas d'infection, de cicatrice, de maladie ou d'affection cutanée telle que candidose, impétigo, psoriasis, eczéma, acné ou dermatite, pellicules, ou plaies ou blessures et/ou autres dermatoses.

L'extrait selon l'invention est un ingrédient topiquement acceptable. On entend par « topiquement acceptable », un ingrédient adapté à une application par voie topique, non toxique, non irritant pour la peau, n'induisant pas de réponse allergique, qui n'est pas instable sur le plan chimique.

L'utilisation de l'extrait peut être par voie orale ou topique. Avantageusement, elle est par voie topique. On entend par « voie topique », l'application locale directe et/ou la vaporisation de l'ingrédient sur la surface de la peau et/ou des muqueuses.

L'extrait peut être appliqué par voie topique sur tout ou partie du corps et/ou du visage, avantageusement choisi parmi les jambes, les pieds, les aisselles, les mains, les cuisses, le ventre, le décolleté, le cou, les bras, le torse, le dos, le visage et/ou le cuir chevelu, plus avantageusement le cuir chevelu et/ou le visage, encore plus avantageusement le visage, et très avantageusement la zone dite « en T » du visage.

Très préférentiellement, la peau n'inclut pas le cuir chevelu.

On entend au sens de l'invention par « diminuer la visibilité des pores de la peau » diminuer le diamètre d'ouverture et/ou l'aire et/ou la densité des pores cutanés.

La visibilité des pores de la peau peut être mise en évidence *in vivo* par une évaluation dite « scoring » par un dermatologique sur une zone prédéfinie après application d'une composition comprenant l'extrait selon l'invention. Elle peut également être mise en évidence par une méthode instrumentale objective par une analyse d'image qui permet d'extraire et de quantifier des paramètres spécifiques des photographies hautes résolution en configuration polarisée croisée du visage des volontaires avant et après application d'une composition comprenant l'extrait selon l'invention. La densité des pores cutanés peut également être mesurée in vivo par imagerie notamment par la technique de projection de franges, en mesurant le paramètre dit de curvature.

On entend par « prévenir l'augmentation de la visibilité des pores de la peau » inhiber l'augmentation du diamètre d'ouverture, notamment la dilatation des pores de la peau, et/ou l'augmentation de l'aire et/ou l'augmentation de la densité des pores cutanés.

La dilatation des pores de la peau peut être induite par des facteurs intrinsèques tels que des variations hormonales, ou des facteurs extrinsèques tels que des températures extérieures élevées ou qui augmentent, les UV, la pollution, des agents agressants chimiques, ou encore l'administration de médicaments modifiant le mécanisme de kératinisation. L'extrait d'*E*. *angustifolium* permet de prévenir la dilatation des pores de la peau.

Dans un mode de réalisation, l'extrait selon l'invention diminue la visibilité des pores de la peau en diminuant l'hyperkératinisation de la peau. On entend par « hyperkératinisation » une hyperprolifération des kératinocytes, sans qu'il s'agisse d'une pathologie nécessitant un traitement pharmaceutique, de façon particulière un traitement dermatologique.

Dans un mode de réalisation préférentiel de l'invention, diminuer l'hyperkératinisation peut signifier augmenter l'expression génique et/ou protéique de l'IGFBP3. Avantageusement ainsi, l'extrait selon l'invention est en quantité efficace pour maintenir et/ou augmenter l'expression génique et/ou protéique de l'IGFBP3 lorsque l'expression d'IGBF3 est augmentée d'au moins 60%, avantageusement d'au moins 80%, encore avantageusement d'au moins 100%, et très avantageusement d'au moins 150% en présence de l'extrait selon l'invention, en comparaison du niveau d'expression détecté en l'absence dudit extrait.

Dans un mode de réalisation avantageux de l'invention, il s'agit d'une augmentation de l'expression protéique de l'IGFB3, préférentiellement mesurée dans des kératinocytes qualifiés de « normaux », c'est-à-dire non pathologiques. Préférentiellement, la mesure de l'expression protéique de l'IGFB3 est effectuée par technique immunohistochimique, avantageusement par technique ELISA.

Encore avantageusement, cette mesure est effectuée dans des kératinocytes normaux cultivés en présence de l'extrait d'*E*. *angustifolium,* de préférence tel que préparé selon l'exemple 1a), très avantageusement encore à une concentration de 0,05% en poids par rapport au volume final du milieu de culture, notamment par la méthode décrite dans les conditions de l'exemple 2a).

Alternativement, diminuer l'hyperkératinisation peut également signifier augmenter l'expression génique et/ou protéique de collagène de type IV. Ainsi l'extrait d'*E*. *angustifolium* selon l'invention est considéré comme en quantité efficace pour maintenir et/ou augmenter l'expression génique et/ou protéique de collagène de type IV lorsque l'expression génique et/ou protéique de collagène de type IV est augmentée d'au moins 40%, préférentiellement d'au moins 80%, encore préférentiellement d'au moins 120%, et très préférentiellement d'au moins 150% en présence de l'extrait selon l'invention, en comparaison du niveau d'expression détecté en l'absence dudit extrait.

Dans un mode de réalisation avantageux de l'invention, il s'agit d'une augmentation de l'expression protéique de collagène de type IV, préférentiellement mesurée dans des kératinocytes qualifiés de « normaux », c'est-à-dire non pathologiques. Préférentiellement, la mesure de l'expression protéique de collagène de type IV est effectuée par technique immunohistochimique.

Encore avantageusement, cette mesure est effectuée dans des kératinocytes normaux cultivés en présence de l'extrait d'*E*. *angustifolium,* de préférence tel que préparé selon l'exemple 1a), avantageusement encore à une concentration de 0,125×10⁻² %, très avantageusement à une concentration de 0,25×10⁻² % en poids par rapport au volume final du milieu de culture, notamment par la méthode décrite dans les conditions de l'exemple 2b). Dans un mode de réalisation préféré, diminuer l'hyperkératinisation signifie augmenter l'expression génique et/ou protéique de l'IGFBP3 et de collagène de type IV.

La mesure de la visibilité des pores de la peau pourra aussi être évaluée *in vivo.* Ainsi encore alternativement, l'extrait d'*E*. *angustifolium* selon l'invention est considéré comme en quantité efficace pour « diminuer la visibilité des pores de la peau et/ou prévenir son augmentation » lorsque l'extrait diminue d'au moins 5%, avantageusement d'au moins 8%, la visibilité de pores de la peau en présence de l'extrait selon l'invention, en comparaison de la visibilité de pores évaluée sans extrait. Dans un mode de réalisation avantageux de l'invention, la mesure de la visibilité des pores est évaluée par un dermatologue spécialiste, au sein d'une population pour laquelle a été appliquée par voie topique sur leur hémi-visage une formulation cosmétique comprenant l'extrait d'*E*. *angustifolium* selon l'invention, la seconde partie du visage servant de contrôle. Avantageusement encore, la formulation cosmétique comprend une concentration finale en poids de 0,20% par rapport au poids total de ladite formulation, de l'extrait testé peutêtre un extrait d'*E*. *angustifolium* selon l'exemple 1a), et il peut être testé dans les conditions décrites dans l'exemple 3.

Encore avantageusement, l'extrait selon la présente invention n'est pas un ingrédient actif contre l'acné ou utilisé comme tel. Autrement dit, il ne diminue pas l'acné et de façon particulière, il n'est pas actif sur la prolifération de *Propionibacterium acnés.*

Diminuer la visibilité des pores permet en outre de prévenir la dilatation des pores de la peau. L'extrait selon l'invention est donc utile pour rendre la peau plus lisse. On entend par « rendre la peau plus lisse » affiner le grain de peau et/ou rendre la peau plus nette.

Dans un mode très préférentiel, l'utilisation de l'extrait selon l'invention n'est pas pour diminuer la visibilité des pores du cuir chevelu et/ou prévenir son augmentation. Encore préférentiellement, l'extrait n'est pas actif sur le cuir chevelu.

L'extrait selon la description peut être un extrait de tout ou partie de la plante *E. angustifolium* choisi parmi les feuilles, la fleur, les pétales, la graine, la tige et/ou les parties aériennes. On entend ici par « parties aériennes » un mélange de feuilles, de fleurs et tiges. L'extrait selon l'invention est un extrait comprenant les parties aériennes et très avantageusement, il s'agit d'un extrait ne contenant que les parties aériennes.

L'extrait selon la description peut être obtenu par différentes méthodes d'extraction connues de l'homme du métier, choisies parmi la macération, la décoction à chaud, par broyage dont le broyage aux ultrasons, à l'aide d'un mixeur, ou encore l'extrait peut être obtenu par extraction dans l'eau, notamment en conditions subcritiques ou supercritiques (dioxyde de carbone). Préférentiellement, l'extraction est réalisée par macération.

L'extraction peut être conduite à partir de matière sèche ou fraîche, avantageusement sèche, en quantité de 0,1 % à 20 % en poids, avantageusement de 1 % à 20 %, très avantageusement de 5 % à 15 %, et encore plus avantageusement de 15 % en poids par rapport au poids total de la matière et du solvant d'extraction.

Au sens de l'invention, on entend par « matière sèche » une matière végétale déshydratée comprenant moins de 15%, avantageusement moins de 10%, encore avantageusement moins de 5% et très avantageusement moins de 1% d'eau.

L'extraction peut être réalisée à une température allant de 4°C à 300°C, y inclus la température ambiante, c'est-à-dire une température de 20°C. Dans un mode préférentiel de réalisation de l'invention, l'extraction sera réalisée à une température de 60°C à 90°C, préférentiellement de 70°C à 85°C, encore préférentiellement à une température de 80°C.

Dans un mode alternatif de réalisation de l'invention, l'extraction est conduite à une température de 4°C à 25°C, encore préférentiellement de 4°C à 20°C, encore avantageusement à température ambiante, c'est-à-dire à 20°C.

Dans encore un autre mode alternatif de réalisation de l'invention, l'extraction est réalisée dans l'eau en conditions subcritiques, à une température allant de 100°C à 300°C, avantageusement de 120°C à 250°C, encore avantageusement à 120°C. L'extraction peut être réalisée à une température donnée unique ou à des températures successives croissantes. Dans un mode de réalisation avantageux de l'invention, l'extraction est réalisée à une température unique de 120°C. Dans un mode alternatif, elle sera conduite selon un gradient de trois températures croissantes comprises entre 100°C et 200°C, tel que 120°C, 140°C puis 160°C ou 110°C, 130°C puis 150°C, ou encore 120°C, 145°C puis 170°C.

On entend par extraction en « conditions subcritiques » une extraction en présence d'eau, dans des conditions de température supérieures à 100°C et de pression inférieure à 221 bars, telle que l'eau reste à l'état liquide mais possède une viscosité et une tension de surface inférieures à celle de l'eau à température ambiante, augmentant sa constante diélectrique.

Ainsi, la pression d'extraction est comprise entre 150 bars et 250 bars, préférentiellement entre 200 et 221 bars, avantageusement dans un autoclave d'extraction sous pression.

L'extraction peut être conduite durant une période de 30 minutes à 24 heures, préférentiellement de 30 minutes à 12 heures, encore préférentiellement durant une période de 1 heure à 5 heures, et encore avantageusement durant une période de 1 heure à 2 heures. Très avantageusement, l'extraction sera conduite durant une période de 2 heures.

L'extrait selon la description peut être obtenu par extraction dans un solvant ou mélange de solvant de préférence un solvant polaire protique, et avantageusement dans l'eau, un alcool, un glycol, un polyol, un mélange eau/alcool, eau/glycol ou eau/polyol (tels que l'eau en mélange avec éthanol, glycérol et/ou butylène glycol et/ou autres glycols tel que xylitol et/ou propanediol, etc.) de 99/1 à 1/99 (p/p), avantageusement dans l'eau comme unique solvant.

De façon particulière, l'extrait selon la description est obtenu par extraction aqueuse. Au sens de la présente description, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau. Dans un autre mode alternatif de réalisation de la description, l'extraction peut être réalisée en présence d'un surfactant non ionique, préférentiellement choisi parmi du lauryl glucoside commercialisé sous le nom de Plantacare^{®} 1200UP par BASF ou encore du caprylyl/capryl glucoside (Plantacare^{®} 810 UP), préférentiellement du caprylyl/capryl glucoside (Plantacare^{®} 810 UP). La concentration en poids du surfactant non ionique pourra être comprise entre 0,5% et 5%, avantageusement entre 0,5 et 1%, encore avantageusement elle sera de 1% en poids par rapport au poids total de l'extrait.

L'extrait selon l'invention est obtenu par extraction dans l'eau comme unique solvant.

Ainsi dans un premier mode avantageux de réalisation de l'invention, l'extrait est obtenu par macération dans l'eau comme unique solvant d'une quantité en poids de 15% de matière sèche des parties aériennes, par rapport au poids total du solvant et de ladite matière, durant une période de 2 heures à température ambiante, c'est-à-dire à une température de 20°C. Le macérat est décanté, centrifugé puis le surnageant est filtré (0,45µm). L'extrait obtenu sous forme liquide est atomisé en présence de maltodextrine (85% p/p) dans les conditions décrites dans l'exemple 1a). L'extrait final se présente sous forme de poudre.

Dans un second mode avantageux de réalisation de l'invention, l'extrait est obtenu par macération dans de l'eau comme solvant unique d'une quantité en poids de 10% de matière sèche des parties aériennes, par rapport au poids total du solvant et de ladite matière, durant une période de 2 heures à température ambiante, c'est-à-dire à une température de 20°C. Le macérat est ensuite décanté, centrifugé puis le surnageant filtré (0,45µm) dans les conditions décrites dans l'exemple 1b). L'extrait final se présente sous forme liquide.

Dans un 3^{ème} mode de réalisation de la description, l'extrait est obtenu par macération dans un mélange eau/éthanol (20:80, v:v) d'une quantité en poids de 15% de matière sèche des parties aériennes, par rapport au poids total du solvant et de ladite matière, durant une période de 1heure à une température de 80°C. Le macérat est décanté, centrifugé puis le surnageant est filtré (0,45µm). L'extrait obtenu sous forme liquide est ensuite atomisé en en présence de maltodextrine (85% p/p) dans les conditions décrites dans l'exemple 1c). L'extrait final se présente sous forme de poudre.

Dans un 4^{ème} mode de réalisation avantageux, l'extrait est obtenu par macération dans de l'eau comme solvant unique d'une quantité en poids de 10% de matière sèche de feuilles séchées, par rapport au poids total du solvant et des feuilles, durant une période de 24 heures à température ambiante, c'est-à-dire à une température de 20°C. Le macérat est décanté, centrifugé puis le surnageant est filtré (0,45µm). L'extrait obtenu sous forme liquide est ensuite atomisé en en présence de maltodextrine (85% p/p) dans les conditions décrites dans l'exemple 1d). L'extrait final se présente sous forme de poudre.

Des étapes supplémentaires de décoloration et/ou désodorisation peuvent être effectuées sur l'extrait à n'importe quel stade de l'extraction et selon les techniques connues par l'Homme du métier. De façon particulière, l'extrait pourra être décoloré au charbon actif.

L'extrait d'E. *angustifolium* obtenu sous forme liquide, notamment dans les conditions décrites dans les exemples 1a) à 1d), peut être ensuite concentré par évaporation du solvant ou séché par exemple par lyophilisation ou par atomisation en présence de maltodextrines. L'extrait se présentera alors sous forme de poudre.

Ainsi dans un mode de réalisation préférentiel de l'invention, l'extrait obtenu sera atomisé en présence d'une concentration en poids de maltodextrines, de préférence une concentration comprise entre 20% et 90%, préférentiellement entre 40 et 85%, encore préférentiellement de 70 à 85% et très avantageusement de 85% en poids, par rapport au poids total de la poudre obtenue. Dans un mode particulier de réalisation de l'invention, notamment pour son utilisation en dermatologie, l'extrait d'*E*. *angustifolium* obtenu est stérilisé. L'extrait selon l'invention peut être utilisé seul sous forme d'ingrédient cosmétique, ou dans une composition cosmétique comprenant au moins un excipient cosmétiquement acceptable. Lorsqu'il est utilisé seul sous forme d'ingrédient cosmétique ou dermatologique, il est préférentiellement solubilisé dans une solution aqueuse contenant de la glycérine, avantageusement présente à une concentration de 60% à 90%, encore avantageusement de 70% à 85%, très avantageusement à une concentration de 80% en poids par rapport au poids total de la solution aqueuse comprenant l'extrait, par exemple tel que présenté dans l'exemple 4.

Dans un mode de réalisation alternatif de réalisation de l'invention, l'extrait sera solubilisé et/ou dilué dans un solvant notamment polaire, tel que l'eau, un alcool, un polyol, un glycol, tel que le pentylène glycol et/ou le butylène glycol et/ou l'hexylène glycol et/ou le caprylyl glycol, ou un de leurs mélanges, préférentiellement un mélange hydroglycolique, encore préférentiellement contenant un glycol choisi parmi l'hexylène glycol, le caprylyl glycol et leurs mélanges. Avantageusement, l'extrait obtenu est dilué et/ou soluble dans une solution aqueuse contenant de l'hexylène glycol, en particulier contenant entre 0,1 et 10 % en poids de l'hexylène glycol, préférentiellement entre 0,5 et 5 % en poids d'hexylène glycol, par rapport au poids total de l'ingrédient cosmétique. Avantageusement, l'extrait obtenu est dilué et/ou soluble dans une solution aqueuse contenant du caprylyl glycol, en particulier contenant entre 0,01 et 5 % en poids de caprylyl glycol, préférentiellement entre 0,1 et 1 % en poids de caprylyl glycol, par rapport au poids total de la solution aqueuse comprenant l'extrait. De façon particulière, la solution aqueuse dans laquelle est solubilisée l'extrait d'*E*. *angustifolium* selon l'invention comprend de la gomme xanthane, en particulier entre 0,01 et 5 % en poids de gomme xanthane par rapport au poids total la solution aqueuse, plus particulièrement entre 0,1 et 1 % en poids de gomme xanthane par rapport au poids total de la solution aqueuse comprenant l'extrait.

De façon avantageuse, la solution dans laquelle est solubilisé l'extrait d'*E*. *angustifolium* selon l'invention comprend de l'hexylène glycol, du caprylyl glycol et de la gomme de xanthane, en particulier dans les quantités indiquées ci-dessus.

L'extrait peut aussi être présent dans une composition cosmétique comprenant en outre au moins un excipient cosmétiquement acceptable. On entend par « acceptable » un excipient cosmétique ou non irritant pour la peau, n'induisant pas de réponse allergique, et stable sur le plan chimique.

Un objet de la présente invention concerne donc l'utilisation de l'extrait d'*E*. *angustifolium* dans une composition cosmétique, pour diminuer la visibilité des pores de la peau et/ou prévenir son augmentation, et/ou pour rendre la peau plus lisse.

Dans un mode de réalisation de l'invention, l'extrait selon l'invention est présent dans la composition cosmétique ou dermatologique à une concentration de 1×10⁻⁴ % à 10 %, préférentiellement de 1×10⁻⁴ % à 5 %, encore préférentiellement de 1×10⁻³ % à 3 % en poids, et très préférentiellement de 1×10⁻³ % à 0,1% par rapport au poids total de la composition. Dans un mode de réalisation très particulier, l'extrait, en particulier celui préparé selon l'exemple 1a), est présent à une concentration de 0,05 % en poids par rapport au poids total de la composition. Dans un autre mode de réalisation très particulier, l'extrait, en particulier celui préparé selon l'exemple 1a), est présent à une concentration de 0,125×10⁻² % ou de 0,25×10⁻² % en poids par rapport au poids total de la composition.

Le ou les excipients pourront être choisis parmi des agents tensioactifs et/ou émulsifiants, des conservateurs, des agents tampons, des agents chélatants, des dénaturants, des agents opacifiants, des ajusteurs de pH, des agents réducteurs, des agents stabilisants, des épaississants, des gélifiants, des polymères filmogènes, des charges, des agents matifiants, des agents de brillance, des pigments, des colorants, des parfums, et leurs mélanges. Le CTFA (Cosmetic Ingrédient Handbook, Second Edition (1992)) décrit différents excipients cosmétiques adaptés à une utilisation dans la présente invention.

Avantageusement, le ou les excipients sont choisis dans le groupe comprenant les polyglycérols, les esters, les polymères et dérivés de cellulose, les dérivés de lanoline, les phospholipides, les lactoferrines, les lactoperoxidases, les stabilisants à base de sucrose, la vitamine E et ses dérivés, les gommes de xanthane, les cires naturelles et synthétiques, les huiles végétales, les triglycérides, les insaponifiables, les phytostérols, les silicones, les hydrolysats de protéines, les bétaïnes, les aminoxides, les extraits de plantes, les esters de saccharose, les dioxydes de titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparabéne, l'éthylparabène, le propylparabène, le butylparabène, le butylène glycol, le caprylyl glycol, les tocophérols naturels, la glycérine, le dihydroxycetyl sodium phosphate, l'isopropyl hydroxycétyl éther, le glycol stéarate, la triisononanoine, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les triglycérides caprique/caprylique, le cétéaryl octanoate, le dibutyl adipate, l'huile de pépins de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, la cire d'abeille, les glycérides d'huile de coeur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée, et leurs mélanges.

La composition cosmétique selon l'invention peut être choisie parmi une solution, aqueuse ou huileuse, une crème ou un gel aqueux ou un gel huileux, notamment un gel douche, un lait, une émulsion, une microémulsion ou une nanoémulsion, notamment huile-dans-eau ou eau-dans huile ou multiple ou siliconée, un masque, un sérum, une lotion, un savon liquide, un pain dermatologique, une pommade, une mousse, un patch, un produit anhydre, de préférence liquide, pâteux ou solide, par exemple sous forme de poudres de maquillage, de bâtonnet ou de stick. De façon avantageuse, il s'agit d'une crème ou d'un sérum.

La composition cosmétique pourra en outre comprendre d'autres ingrédients actifs cosmétiques. De nombreux ingrédients cosmétiquement actifs sont connus par l'homme du métier pour améliorer la santé et/ou l'apparence physique de la peau. D'autre part, les composés décrits dans la présente invention peuvent avoir un effet de synergie lorsqu'ils sont combinés les uns aux autres. Ces combinaisons sont également couvertes par la présente invention. Le CTFA Cosmetic Ingrédient Handbook, Second Edition (1992) décrit différents ingrédients cosmétiques et pharmaceutiques utilisés couramment dans l'industrie cosmétique et pharmaceutique, qui sont en particulier adaptés à une utilisation topique. Des exemples de ces classes d'ingrédients comprennent, sans en être limité les composés suivants: abrasif, absorbants, composé à but esthétique tel que les parfums, les pigments, les colorants, les huiles essentielles, les astringents, par exemple l'huile de clou de girofle, menthol, camphre, l'huile d'eucalyptus, eugénol, menthyl lactate, distillat d'hamélis, les agents anti-acnés, les agents anti-floculants, les agents antimousse, les agents antimicrobiens (par exemple: iodopropyl butylcarbamate), les antioxydants, les liants, les additives biologiques, les agents tampon, les agents gonflants, les agents chélatants, les additifs, les agents biocides, les dénaturants, les épaississants, et les vitamines, et les dérivés ou équivalents de ceux-ci, les matériaux formant des films, les polymères, les agents opacifiants, les ajusteurs de pH, les agents réducteurs, les agents dépigmentants ou éclaircissants (par exemple : hydroquinone, acide kojique, acide ascorbique, magnésium ascorbyl phosphate, ascorbyl glucosamine), les agents de conditionnement (par exemple : les humectants).

De manière avantageuse, l'extrait d'*E*. *angustifolium* selon l'invention peut être utilisé, éventuellement dans une composition cosmétique ou pharmaceutique, avantageusement dermatologique, comme seul ingrédient actif ou en combinaison avec un ou plusieurs autres agents actifs à effet complémentaire choisi parmi :
- un agent séborégulateur cosmétique et/ou dermatologique, préférentiellement, un extrait de *Bixa orellana,* la sarcosine tel que commercialisé par la Demanderesse sous le nom MAT-XS ^{™} Clinical, un extrait *d'Orthosiphon stamineus* tel que commercialisé par la Demanderesse sous le nom MAT-XS ^{™} Bright, le gluconate de zinc, le salicylate de zinc, l'acide azélaïque et/ou leurs dérivés, et/ou leurs mélanges, avantageusement en combinaison avec le gluconate de zinc, et/ou un agent exfoliant et/ou kératolytique : les alpha-hydroxy-acides (AHA) notamment l'acide salicylique, éventuellement en combinaison avec des protéines d'acacia, l'acide malique, éventuellement en association avec des protéines d'amande, l'acide glycolique ; l'acide lactique et/ou leurs dérivés; et/ou leurs mélanges,
- un agent absorbeur de sébum: un talc et/ou un polymère absorbant,
- un agent ayant une action sur le microbiote notamment cutané tel qu'un extrait de *Peumus boldus,* notamment celui commercialisé par la Demanderesse sous le nom de Betapur^{™} et/ou un agent antibiotique local, en particulier l'érythromycine et/ou le phosphate de clindamycine ; les agents équilibrants la flore microbienne tels qu'un mélange de pullulan, alginate de sodium et hyaluronate de sodium commercialisé par la Demanderesse sous le nom PatcH20 ^{™} et notamment associé à la sérine, tréhalose et urée et décrit dans la demande de brevet WO2014027163A2 ; et/ou un extrait de *Saccharomyces cerevisiae* pour augmenter la flore microbienne commensale cutanée et/ou mucosale commercialisé par la Demanderesse sous le nom Relipidium^{™},
- un agent comédolytique tel que l'acide rétinoique et/ou un de ses dérivés tels que isotrétinoine, adapalène et/ou l'acide 13-cis-rétinoique et le péroxyde de benzoyle;
- un agent hydratant tel qu'un polysaccharide extrait de graines de *Cassia angustifolia* commercialisée sous le nom de Hyalurosmooth^{™} par la demanderesse, ou un agent choisi parmi une des combinaisons contenant du pullulan, du hyaluronate de sodium et de l'alginate de sodium commercialisé sous le nom de PatcH2O^{™} par la demanderesse ou encore un ou plusieurs des composés du facteur natural d'hydratation (Natural Moisturizing Factor) ou un extrait naturel de miel commercialisé par la demanderesse sous le nom de Melhydran^{™} et/ou un composé de la famille des glucosyl glycérides, en particulier l'hexosyl glycéride, un extrait de péricarpe de *Litchi chinensis* sous le nom Litchiderm^{™} par la déposante.

Avantageusement, la composition cosmétique contenant l'extrait selon l'invention contiendra un autre agent séborégulateur choisi parmi la sarcosine et un extrait *d'Orthosiphon stamineus,* un extrait de *Bixa orellana*, le gluconate de zinc et leurs mélanges, un agent hydratant choisi parmi un extrait de graines de *Cassia angustifolia*, l'hexosyl glycéride, un mélange contenant du pullulan, du hyaluronate de sodium et de l'alginate de sodium et leurs mélanges. De manière avantageuse, la composition contenant l'extrait selon l'invention contiendra en outre un agent agissant sur la flore bactérienne choisi parmi un extrait de *Peumus boldus,* un mélange de pullulan, alginate de sodium et hyaluronate de sodium, un extrait de *Saccharomyces cerevisiae* et leurs mélanges.

La composition cosmétique comprenant l'extrait selon l'invention peut comprendre les ingrédients actifs cosmétiques bien connus des compositions cosmétiques choisis parmi un agent stimulant la synthèse de fibronectine, en particulier un extrait de mais, un tel extrait étant notamment commercialisé par la Demanderesse sous le nom Deliner^{™}, un agent de protection du facteur de croissance des fibroblastes (FGF2) de la matrice extracellulaire contre sa dégradation et/ou sa dénaturation, notamment un extrait d'*Hibiscus Abelmoscus* tel que décrit dans la demande de brevet au nom de la Demanderesse déposée sous le numéro FR0654316 et/ou un agent de stimulation de croissance des fibroblastes par exemple un extrait de soja fermenté contenant des peptides, connu sous le nom de Phytokine^{™} commercialisé par la Demanderesse et également décrit dans la demande de brevet EP1119344 B1 (Laboratoires Expanscience), et préférentiellement une combinaison de ces deux extraits, un agent stimulant la synthèse de laminine, en particulier un extrait de malt modifié par biotechnologie, un tel extrait étant notamment commercialisé par la Demanderesse sous le nom Basaline^{™} ; un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (*Alpinia galanga),* un agent stimulant la synthèse de lysyl oxydase like (LOXL) tel que ceux décrits dans la demande de brevet FR2855968, et en particulier un extrait *d'Anethum graveolens,* un ou plusieurs agents anti-pollution tels qu'un extrait de feuilles d'*Argania spinosa* commercialisé sous le nom d'Arganyl^{™} ou un extrait de graines de *Moringa oleifera* commercialisé sous le nom de Purisoft^{™} par la demanderesse ou encore un extrait de racine d'*Eperua falcata* commercialisé sous le nom de Eperuline^{™}, un agent stimulant la synthèse d'ATP intracellulaire, notamment un extrait d'algue *Laminaria digitata*, un agent à action globale anti-âge, notamment anti-tâches pigmentaires en particulier la niacinamide ou vitamine B3, et l'un quelconque de leurs mélanges.

Un autre objet de l'invention concerne un procédé de soin cosmétique comprenant l'administration par voie topique ou orale, avantageusement l'application par voie topique, de l'extrait selon l'invention ou d'une composition cosmétique le comprenant, pour diminuer la visibilité des pores de la peau et/ou prévenir son augmentation. Ledit procédé permet ainsi de rendre la peau plus lisse.

Dans un mode de réalisation de l'invention, le procédé de soin cosmétique comprend l'application par voie topique de l'extrait selon l'invention ou d'une composition cosmétique le comprenant sur tout ou partie du corps et/ou du visage, avantageusement choisi parmi les jambes, les pieds, les aisselles, les mains, les cuisses, le ventre, le décolleté, le cou, les bras, le torse, le dos, le visage et/ou le cuir chevelu, plus avantageusement le cuir chevelu et/ou le visage, encore plus avantageusement le visage.

Des exemples faisant référence à la description sont présentés ci-après. Ces exemples sont donnés à titre d'illustration et ne sauraient limiter en aucun cas la portée de l'invention. Chaque exemple a une portée générale. Les exemples font partie intégrante de la présente invention et toute caractéristique apparaissant nouvelle par rapport à un état de la technique antérieure quelconque à partir de la description prise dans son ensemble, y compris les exemples, fait partie intégrante de l'invention.

Sauf indication contraire, la température est exprimée en degré Celsius (°C), l'abréviation « p » signifie poids et l'abréviation « v » signifie volume.

### Exemples

La matière sèche servant à préparer l'extrait selon l'invention provient de France.

### Exemple 1) : Préparation d'un extrait d'E. angustifolium selon l'invention

Exemple 1a) Une quantité en poids de 15% de matière sèche des parties aériennes, par rapport au poids total du solvant et de ladite matière, a été macérée dans de l'eau comme solvant unique durant une période de 2 heures à température ambiante, c'est-à-dire à une température de 20°C. Le macérat a été décanté, centrifugé puis le surnageant filtré (0,45µm). L'extrait obtenu sous forme liquide a ensuite été atomisé en présence de maltodextrine (85% p/p). L'extrait final se présente sous forme de poudre.

Exemple 1b) Une quantité en poids de 10% de matière sèche des parties aériennes, par rapport au poids total du solvant et de ladite matière, a été macérée dans de l'eau comme solvant unique durant une période de 2 heures à température ambiante, c'est-à-dire à une température de 20°C. Le macérat a été décanté, centrifugé puis le surnageant filtré (0,45µm). L'extrait obtenu se présente sous forme liquide.

Exemple comparatif 1c) Une quantité en poids de 15% de matière sèche des parties aériennes, par rapport au poids total du solvant et de ladite matière, a été macérée dans un mélange eau/éthanol (20: 80, v: v) durant une période de 1heure à une température de 80°C. Le macérat a été décanté, centrifugé puis le surnageant filtré (0,45µm). L'extrait obtenu sous forme liquide a ensuite été atomisé en présence de maltodextrine (85% p/p). L'extrait final se présente sous forme de poudre.

Exemple 1d) Une quantité en poids de 10% de matière sèche de feuilles, par rapport au poids total du solvant et des feuilles, a été macérée dans de l'eau comme solvant unique durant une période de 24 heures à température ambiante, c'est-à-dire à une température de 20°C. Le macérat a été décanté, centrifugé puis le surnageant filtré (0,45µm). L'extrait obtenu sous forme liquide a ensuite été atomisé en présence de maltodextrine (85% p/p). L'extrait final se présente sous forme de poudre.

### Exemple 2) : Diminution de la visibilité des pores de la peau

### Exemple 2a) : Augmentation de l'expression protéique d'IGFBP3

### Protocole:

Des kératinocytes « normaux », c'est-à-dire non pathologiques, provenant d'un donneur sain âgé de 19 ans, ont été cultivés dans un milieu défini (KSFM) jusqu'à confluence à une température de 37°C (Atmosphère CO₂ 5%) puis l'extrait d'*E*. *angustifolium* a été ajouté au milieu à des concentrations de 5×10⁻³ % et 2,5×10⁻² % en poids par rapport au volume final du milieu. Le même milieu sans ajout d'extrait a servi de contrôle. Les surnageants des milieux de culture ont été récupérés par centrifugation puis l'expression protéique de l'IGFB3 a été mesurée par technique ELISA (LSBio Human IGFBP-3 ELISA Kit ref LS-F24538). La densité optique a été lue à 450nm. Les résultats ont été normalisés par rapport au contrôle et représentent la moyenne (MOY) de 6 expériences (n=6) (ET : Ecart-type).

### Résultats

**Tableau I**

| | MOY | ET |
|---|---|---|
| Contrôle | 100 | 28 |
| Extrait d'*E*. *angustifolium* 5 x 10⁻³ % (p/v) selon l'exemple 1a) | 269 | 54 |
| Extrait d'*E*. *angustifolium* 2,5 x 10⁻² % (p/v) selon l'exemple 1a) | 619 | 55 |

*Conclusion:* les résultats ont montré une augmentation de l'expression protéique de l'IGFB3 dans les kératinocytes, signifiant une diminution de l'hyperkératinisation, donc la capacité de l'extrait d'*E*. *angustifolium* selon l'invention à diminuer la visibilité des pores de la peau et/ou prévenir son augmentation.

### Exemple 2b) : Augmentation de l'expression protéique de collagène de type IV

### Protocole:

Des kératinocytes humains dits « normaux », c'est-à-dire ne présentant pas de pathologie, issus d'une donneuse saine de 24 ans ont été cultivés dans un milieu défini (KSFM) durant une période de 48 heures en présence de différentes concentrations finales d'*E*. *angustifolium,* puis le milieu cellulaire éliminé. Le même milieu de culture sans ajout d'extrait selon l'invention a été utilisé comme témoin (Contrôle). Le tapis cellulaire obtenu a été lysé avec une solution d'hydroxyde d'ammonium, puis le dosage du collagène de type IV a été effectué avec un anticorps anti-collagène IV, dilué au 1/4000 dans une solution tampon (PBS). Après une période de 90 minutes, un anticorps secondaire dilué au 1/25000 a été appliqué durant une période de 60 minutes. Après lavage, une solution de révélation a été ajoutée et la fluorescence a été mesurée (ENVision, PerkinElmer). Les résultats de fluorescence ont été normalisés par rapport à la fluorescence obtenue avec le même milieu cellulaire en l'absence de l'extrait *d'Epilobium angustifolium* (Contrôle) et ont été rapportés à la quantité d'ADN obtenue dans chaque condition. Les résultats présentés correspondent à la moyenne de 6 essais (n=6) (ET : Ecart-type).

### Résultats :

**Tableau II**

| | MOY | ET |
|---|---|---|
| Contrôle | 100,0 | 7,5 |
| Extrait d'*E*. *angustifolium* 0,125x 10⁻² % (p/v) selon l'exemple 1a) | 181,7 | 27,2 |
| Extrait d'*E*. *angustifolium* 0,25x 10⁻² % (p/v) selon l'exemple 1a) | 244,7 | 13,5 |

*Conclusion :* l'extrait d'*E*. *angustifolium* a augmenté l'expression protéique du collagène de type IV dans les kératinocytes analysés, démontrant sa capacité à diminuer la visibilité des pores de la peau et/ou prévenir son augmentation.

### Exemple 3) : Analyse de la diminution de la visibilité des pores de la peau par mesure de l'aire des pores de la peau

*Protocole :* Une formulation cosmétique comprenant l'extrait d'*E*. *angustifolium* tel que préparé dans l'exemple 1a) à une concentration finale en poids de 0,20 % par rapport au poids total de la formulation cosmétique, a été appliquée 2 fois par jour durant une période de 56 jours sur l'hémi-visage d'une population humaine de 34 personnes de sexe féminin d'origine asiatique et âgées de 20 à 45 ans. La même formulation cosmétique comprenant de l'eau à la place de l'extrait d'*E*. *angustifolium* (Contrôle) a été appliquée dans les mêmes conditions sur l'autre hémi-visage de la même population. Les mesures ont été effectuées aux temps D0 et D56 (56jours). Un spécialiste du domaine, c'est-à-dire un dermatologue, a évalué les hémi-visages des 34 personnes sur lesquels la formulation cosmétique a été appliquée, en comparaison de ceux sur lesquels a été appliquée la formulation contrôle. Les résultats de mesure de l'aire des pores cutanés figurent dans le tableau III ci-dessous.

### Résultats :

**Tableau III**

| | MOY D0 | MOY D56/D0 |
|---|---|---|
| Contrôle | 100 | 97* |
| Extrait d'*E*. *angustifolium* 0,20% (p/v) | 100 | 91* |

| | | |
|---|---|---|
| *Test de Wilcoxon (p<0,001) | | |

*Conclusion* : l'analyse a montré une diminution moyenne de l'aire des pores de 9% en présence de l'extrait d'*E. angustifolium* au bout de 56 jours d'application bi-quotidienne.

### Exemple 4) : Ingrédient cosmétique comprenant un extrait d'E. angustifolium selon l'invention

Les pourcentages sont exprimés en poids.

| | |
|---|---|
| Extrait d'*E*. *angustifolium* Ex. 1a) | 0,2% |
| Eau | 19,8% |
| Glycérine | 80% |

### Exemple 5) : Formulation cosmétique comprenant un extrait d'E. angustifolium selon l'invention

L'extrait utilisé est celui obtenu dans l'exemple 1a). Les pourcentages sont exprimés en poids par rapport au poids total de la formulation.

**Tableau IV**

| Phase | | % |
|---|---|---|
| A | Eau | 88,80 |
| A | Propylène glycol, phénoxyéthanol, chlorophénésine, méthylparabène | 1,00 |
| A | Polyacrylate de sodium | 1,00 |
| B | Huile végétale, glycérine, glucoside de lauryle, oléate de glycéryl, polyglycéryl-2-dipolyhydrostéarate, carbonate de dicaprylyle | 3,00 |
| B | Caprylate de propylheptyle | 5,00 |
| C | Extrait d'*E*. *angustifolium* Ex. 1a) (Maltodextrines 85%) | 0,20 |
| C | Eau osmosée | 1,00 |

## Revendications

1. Utilisation cosmétique non thérapeutique d'un extrait des parties aériennes d'*Epilobium angustifolium* pour diminuer la visibilité des pores de la peau et/ou prévenir l'augmentation de la visibilité des pores de la peau, **caractérisé en ce que** l'extrait est obtenu dans l'eau comme unique solvant.

2. Utilisation cosmétique selon la revendication 1, pour diminuer la visibilité des pores de la peau.

3. Utilisation cosmétique selon la revendication 1 ou 2 **caractérisée en ce que** l'extrait maintient et/ou augmente l'expression génique et/ou protéique d'IGFBP3 et/ou de collagène de type IV.

4. Utilisation cosmétique selon l'une quelconque des revendications 1 à 3, **caractérisée en ce que** l'extrait rend la peau plus lisse.

5. Utilisation cosmétique selon l'une quelconque des revendications 1 à 4, **caractérisée en ce que** l'extrait est compris dans une composition cosmétique comprenant au moins un excipient cosmétiquement acceptable, à une concentration de 1×10⁻⁴ % à 10 %, préférentiellement de 1×10⁻⁴ % à 5 %, et encore préférentiellement de 1×10⁻³ % à 3 % en poids par rapport au poids total de la composition.

6. Utilisation cosmétique selon la revendication 5, **caractérisée en ce que** la composition cosmétique est appliquée sur tout ou partie du corps et/ou du visage choisie parmi les jambes, les pieds, les aisselles, les mains, les cuisses, le ventre, le décolleté, le cou, les bras, le torse, le dos, le visage, plus avantageusement le visage.

7. Procédé de soin cosmétique pour diminuer la visibilité des pores de la peau et/ou prévenir l'augmentation de la visibilité des pores de la peau, comprenant l'administration par voie topique ou orale, préférentiellement l'application par voie topique, d'un extrait des parties aériennes d'*E. angustifolium* ou d'une composition cosmétique le comprenant, **caractérisé en ce que** l'extrait est obtenu dans l'eau comme unique solvant.

8. Procédé de soin cosmétique selon la revendication 7, **caractérisé en ce qu'**il rend la peau plus lisse.

9. Procédé de soin cosmétique selon la revendication 7 ou 8 **caractérisé en ce qu'**il comprend l'application par voie topique de l'extrait d'*E. angustifolium* ou d'une composition cosmétique le comprenant sur tout ou partie du corps et/ou du visage choisi parmi les jambes, les pieds, les aisselles, les mains, les cuisses, le ventre, le décolleté, le cou, les bras, le torse, le dos, le visage, plus avantageusement le visage.

## Patentansprüche

1. Nichttherapeutische kosmetische Verwendung eines Extrakts der oberirdischen Teile von *Epilobium angustifolium* zur Verringerung der Sichtbarkeit der Hautporen und/oder zur Verhinderung der Zunahme der Sichtbarkeit der Hautporen, **dadurch gekennzeichnet, dass** der Extrakt in Wasser als einzigem Lösungsmittel erhalten wird.

2. Kosmetische Verwendung nach Anspruch 1 zur Verringerung der Sichtbarkeit der Hautporen.

3. Kosmetische Verwendung nach Anspruch 1 oder 2, **dadurch gekennzeichnet, dass** der Extrakt die Genexpression und/oder Proteinexpression von IGFBP3 und/oder die Expression von Kollagen Typ IV aufrechterhält und/oder erhöht.

4. Kosmetische Verwendung nach einem der Ansprüche 1 bis 3, **dadurch gekennzeichnet, dass** der Extrakt die Haut glatter macht.

5. Kosmetische Verwendung nach einem der Ansprüche 1 bis 4, **dadurch gekennzeichnet, dass** der Extrakt in einer kosmetischen Zusammensetzung, die mindestens einen kosmetisch unbedenklichen Hilfsstoff umfasst, in einer Konzentration von 1×10⁻⁴ bis 10 Gew.-%, bevorzugt von 1×10⁻⁴ bis 5 Gew.-% und weiter bevorzugt von 1×10⁻³ bis 3 Gew.-%, bezogen auf das Gesamtgewicht der Zusammensetzung, enthalten ist.

6. Kosmetische Verwendung nach Anspruch 5, **dadurch gekennzeichnet, dass** die kosmetische Zusammensetzung auf die Gesamtheit oder einen Teil des Körpers und/oder des Gesichts, ausgewählt aus Beinen, Füßen, Achselhöhlen, Händen, Oberschenkeln, Bauch, Dekolleté, Hals, Armen, Rumpf, Rücken, Gesicht, noch vorteilhafterweise Gesicht, aufgebracht wird.

7. Kosmetisches Pflegeverfahren zur Verringerung der Sichtbarkeit der Hautporen und/oder zur Verhinderung der Zunahme der Sichtbarkeit der Hautporen, umfassend die topische oder orale Verabreichung, vorzugsweise die topische Applikation, eines Extrakts der oberirdischen Teile von *E*. *angustifolium* oder einer kosmetischen Zusammensetzung, die diesen umfasst, **dadurch gekennzeichnet, dass** der Extrakt in Wasser als einzigem Lösungsmittel erhalten wird.

8. Kosmetisches Pflegeverfahren nach Anspruch 7, **dadurch gekennzeichnet, dass** es die Haut glatter macht.

9. Kosmetisches Pflegeverfahren nach Anspruch 7 oder 8, **dadurch gekennzeichnet, dass** es die topische Applikation des Extrakts von *E*. *angustifolium* oder einer kosmetischen Zusammensetzung, die diesen umfasst, auf die Gesamtheit oder einen Teil des Körpers und/oder des Gesichts, ausgewählt aus Beinen, Füßen, Achselhöhlen, Händen, Oberschenkeln, Bauch, Dekolleté, Hals, Armen, Rumpf, Rücken, Gesicht, noch vorteilhafterweise Gesicht, umfasst.

## Claims

1. Non-therapeutic cosmetic use of an extract of the aerial parts of *Epilobium angustifolium* for reducing the visibility of the pores of the skin and/or preventing the increase in the visibility of the pores of the skin, **characterized in that** the extract is obtained in water as sole solvent.

2. Cosmetic use according to Claim 1, for reducing the visibility of the pores of the skin.

3. Cosmetic use according to Claim 1 or 2, **characterized in that** the extract maintains and/or increases the gene and/or protein expression of IGFBP3 and/or of type IV collagen.

4. Cosmetic use according to any one of Claims 1 to 3, **characterized in that** the extract makes the skin smoother.

5. Cosmetic use according to any one of Claims 1 to 4, **characterized in that** the extract is included in a cosmetic composition comprising at least one cosmetically acceptable excipient, at a concentration of from 1 × 10⁻⁴% to 10%, preferentially from 1 × 10⁻⁴% to 5%, and more preferentially from 1 × 10⁻³% to 3% by weight, relative to the total weight of the composition.

6. Cosmetic use according to Claim 5, **characterized in that** the cosmetic composition is applied to all or part of the body and/or of the face, chosen from the legs, feet, underarms, hands, thighs, stomach, neckline, neck, arms, torso, back, face, and more advantageously the face.

7. Cosmetic care process for reducing the visibility of the pores of the skin and/or preventing the increase in the visibility of the pores of the skin, comprising the topical or oral administration, preferentially the topical application, of an extract of the aerial parts of E. *angustifolium* or of a cosmetic composition including the extract, **characterized in that** the extract is obtained in water as sole solvent.

8. Cosmetic care process according to Claim 7, **characterized in that** it makes the skin smoother.

9. Cosmetic care process according to Claim 7 or 8, **characterized in that** it comprises the topical application of the E. *angustifolium* extract or of a cosmetic composition including the extract to all or part of the body and/or of the face, chosen from the legs, feet, underarms, hands, thighs, stomach, neckline, neck, arms, torso, back, face, and more advantageously the face.
